# EUROPÄISCHE PATENTSCHRIFT

(11) **EP 0 582 247 B1**
(45) Veröffentlichungstag und Bekanntmachung des Hinweises auf die Patenterteilung: **08.05.1996**
(21) Anmeldenummer: 93112348.3
(22) Anmeldetag: 02.08.1993
(51) Int. Cl.: C07C 303/32, C07C 309/58

(54) **Verfahren zur Herstellung von Alkalisalzen der 3-Sulfobenzoesäure**
Method for the preparation of alkali metal salts of 3-sulphobenzoic acid
Procédé pour la préparation de sels de métaux alcalins d'acide 3-sulfobenzoiques

(30) Priorität: 07.08.1992 DE 4226130
(43) Veröffentlichungstag der Anmeldung: 09.02.1994
(73) Patentinhaber: HOECHST AKTIENGESELLSCHAFT, D-65926 Frankfurt am Main (DE)
(72) Erfinder: Meier, Michael, Dr., D-60487 Frankfurt am Main (DE)

(56) Entgegenhaltungen:
- HELVETICA CHIMICA ACTA, Bd. 24, Nr. 2, 15. März 1941, Basel, CH, Seiten 197 - 212 P. RUGGLI, ET AL.: 'Die Säurechloride der m-Sulfo-benzoesäure und ihre Umsetzungen mit Aminen und Phenolen'
- J. MARCH: 'ADVANCED ORGANIC CHEMISTRY, 3rd Edition,' 1985 , JOHN WILEY & SONS , NEW YORK, US

## Beschreibung

Die vorliegende Erfindung betrifft ein sich gegenüber dem Stand der Technik durch einen verringerten Anfall von Abwasser auszeichnendes Verfahren zur Herstellung von Alkalisalzen der 3-Sulfobenzoesäure.

Es ist aus Offermann, Liebigs Annalen der Chemie 280, 6 (1894) bekannt, das Natriumsalz der 3-Sulfobenzoesäure durch Sulfonierung von Benzoesäure mit Oleum bei 200°C, Eintragen des resultierenden Reaktionsproduktes in ein Wasser-Eis-Gemisch und Zusatz von Natriumchlorid herzustellen. Nachteilig an diesem Verfahren ist, daß man eine Natriumchlorid enthaltende, verdünnte Schwefelsäure erhält, die technisch nur unter großem Aufwand aufgearbeitet werden kann. Aufgrund des hohen Natriumchlorid-Gehaltes enthält das Reaktionsprodukt lediglich 75 % des Natriumsalzes der 3-Sulfobenzoesäure.

Nach dem in der US 4 358 410 beschriebenen Verfahren wird Benzoesäure mit Oleum bei 130°C umgesetzt und das anfallende Reaktionsgemisch anschließend mit einer wäßrigen, konzentrierten Natriumchloridlösung versetzt und abgekühlt. Dies führt zwar zu einer verbesserten Reinheit des Produktes, aber auch zu einem großen Anteil schwer zu entsorgender, verdünnter Schwefelsäure, die mit Natriumchlorid verunreinigt ist.

Nach einem von Ruggli und Grün in Helv. Chim. Acta 24, 197 (1941) beschriebenen Verfahren erhält man freie 3-Sulfobenzoesäure durch Umsetzung von 3-Benzoesäuresulfochlorid mit der 10-fachen Menge Wasser unter Erwärmen. Anschließend wird das Wasser abdestilliert, wobei ein Sirup erhalten wird. Man versetzt diesen Sirup mit frischem Wasser und destilliert das Wasser anschließend wieder ab. Dieser Vorgang wird zweimal wiederholt. Der Nachteil dieses Verfahrens besteht darin, daß dreimal große Mengen Wasser, die nicht wieder verwendet werden können, abdestilliert werden müssen. Dies erfordert einen sehr hohen Energiebedarf. Zudem ist eine technische Realisierung nur unter Schwierigkeiten möglich, da im Reaktionsgefäß 3-Sulfobenzoesäure zurückbleibt, die als Festprodukt bei 150°C schmilzt und demzufolge nur als heiße Schmelze aus dem Reaktionsgefäß abgelassen werden kann. Die DE 3 122 264 und Ind. Eng. Chem. 45, 2065 (1953) betreffen ein Verfahren zur Herstellung von 3-Sulfobenzoesäure durch Umsetzung von Benzoesäure mit einer stöchiometrischen Menge Schwefeltrioxid. Hierbei verbleibt jedoch ebenfalls ein Festprodukt im Reaktor, das nur in schmelzflüssige Form abgelassen werden kann. Dies zieht einen erhöhten technischen Aufwand nach sich und ergibt Probleme bei der Weiterverarbeitung. Zudem ist die Handhabung von Schwefeltrioxid nicht unproblematisch und erfordert besondere Vorkehrungen.

Es besteht daher ein Bedarf nach einem Verfahren, das sich nicht nur technisch leicht durchführen läßt, sondern auch zu einer Verringerung der Abwassermenge führt. Es soll darüber hinaus sicherstellen, daß das gewünschte Wertprodukt sowohl in hoher Ausbeute als auch in großer Reinheit anfällt.

Gelöst wird diese Aufgabe durch ein Verfahren zur Herstellung von Alkalisalzen der 3-Sulfobenzoesäure. Es ist dadurch gekennzeichnet, daß man 3-Benzoesäuresulfochlorid mit einer wäßrigen Alkalichloridlösung versetzt, das Gemisch erhitzt, nach Umsetzung auf ≦ 15°C abkühlt und das auskristallisierte Alkalisalz der 3-Sulfobenzoesäure abtrennt.

Das Einsatzmaterial, 3-Benzoesäuresulfochlorid läßt sich nach dem beispielsweise in der DE 3 919 840 beschriebenen Verfahren herstellen. Es kann vorteilhafterweise in wasserfeuchtem Zustand eingesetzt werden. Sein Wassergehalt beträgt üblicherweise 20 bis 40 Gew.-%.

Als Alkalichlorid setzt man Natriumchlorid oder Kaliumchlorid, insbesondere Natriumchlorid ein.

Die wäßrige Lösung soll das Alkalichlorid in ausreichender Menge aufweisen. Üblicherweise verwendet man eine wäßrige Lösung mit 15 bis 30, insbesondere 18 bis 28, bevorzugt 22 bis 27 Gew.-% Alkalichlorid.

Gebraucht man Natriumchlorid als Alkalichlorid, so verwendet man üblicherweise eine wäßrige Lösung, die 22 bis 26,4 Gew.-% Natriumchlorid enthält. Lösungen mit einem niedrigeren Natriumchlorid-Gehalt können auch verwendet werden, ziehen aber eine entsprechend verringerte Ausbeute an 3-Sulfobenzoesäure Natriumsalz sowie gegebenenfalls einen entsprechend erhöhten Anteil von Abwasser nach sich. Höher konzentrierte Natriumchlorid-Lösungen können aufgrund der Löslichkeit von Natriumchlorid in Wasser eventuell zu Schwierigkeiten in der Handhabung führen.

Die Menge an wäßriger Alkalichlorid-Lösung richtet sich auch in gewissem Umfange nach der Alkalichlorid-Konzentration der Lösung.

Üblicherweise setzt man 0,5 bis 10, insbesondere 0,7 bis 4, bevorzugt 0,8 bis 2,5 Gew.-Teile wäßrige Alkalichlorid-Lösung, insbesondere Natriumchlorid-Lösung, je Gew.-Teil 3-Benzoesäuresulfochlorid ein.

Man vermischt das 3-Benzoesäuresulfochlorid mit der wäßrigen Alkalichlorid-Lösung intensiv und erwärmt das resultierende Gemisch. Im allgemeinen genügt es, eine Reaktionstemperatur von 80°C einzustellen. Dies gewährleistet einen ausreichend schnellen Reaktionsverlauf. Es hat sich als nützlich erwiesen, eine Reaktionstemperatur zwischen 80°C und der jeweiligen Siedetemperatur des Reaktionsgemisches, insbesondere 90°C und der jeweiligen Siedetemperatur des Reaktionsgemisches einzustellen. Es lassen sich auch tiefere Temperaturen anwenden, man muß jedoch dabei in Kauf nehmen, daß die Reaktionszeiten sich verlängern. Höhere Temperaturen begünstigen zwar die Geschwindigkeit der Umsetzung, erfordern jedoch unter Umständen eine Durchführung der Reaktion unter Druck.

Nach Beendigung der Reaktion kühlt man das Reaktionsgemisch ab. Die Endtemperatur des Abkühlvorganges soll ≦ 15°C betragen. Man kann den Abkühlvorgang in einem Schritt oder verteilt auf zwei Schritte durchführen.

Es hat sich herausgestellt, daß es häufig zweckmäßig ist, den Abkühlvorgang in zwei Schritten durchzuführen, wobei zunächst in einer ersten Stufe eine Temperatur von 60 bis 40°C, insbesondere 50 bis 40°C eingestellt wird und anschließend auf die Endtemperatur ≦ 15°C abgekühlt wird. Bedingt durch die Löslichkeit des Alkalisalzes der 3-Sulfobenzoesäure in Wasser bestimmt die Endtemperatur des Kühlvorganges in gewissem Umfange auch die Wertprodukt-Ausbeute. Um die Ausbeute günstig zu beeinflussen, empfiehlt es sich eine Temperatur unterhalb von 15, insbesondere ≦ 10, bevorzugt ≦ 50°C zu wählen.

Nach Beendigung des Abkühlens trennt man das auskristallisierte Alkalisalz der 3-Sulfobenzoesäure, insbesondere das Natriumsalz der 3-Sulfobenzoesäure, beispielsweise mittels Filtration ab. Zur weiteren Reinigung empfiehlt es sich, das abgetrennte Kristallisat mit einer wäßrigen Alkalichlorid-Lösung zu waschen. Dabei verwendet man eine wäßrige Kaliumchloridlösung, um das Kaliumsalz der 3-Sulfobenzoesäure zu waschen und eine Natriumchlorid-Lösung, um das Natriumsalz der 3-Sulfobenzoesäure nachzubehandeln.

Die wäßrige Alkalisalz-Waschlösung enthält üblicherweise 1 bis 10, insbesondere 3 bis 8 Gew.-% Alkalichlorid, vorzugsweise Natriumchlorid. Die Lösung wird zweckmäßigerweise auf eine Temperatur ≦ 15, insbesondere ≦ 10, bevorzugt ≦ 5°C gekühlt, ehe sie für den Waschvorgang eingesetzt wird.

Die einzusetzende Menge Waschlösung richtet sich in gewissem Umfange nach der Konzentration der verwendeten wäßrigen Alkalichloridlösung und deren Temperatur. Es hat sich gezeigt, daß üblicherweise 0,3 bis 2,5, insbesondere 0,5 bis 2 Gew.-Teile Waschlösung je Gewichtsteil ursprünglich eingesetztes 3-Benzoesäuresulfochlorid genügen.

Es ist überraschend, daß sich bei dem erfindungsgemäßen Verfahren das Alkalisalz der 3-Sulfobenzoesäure direkt durch Umsetzung von 3-Benzoesäuresulfochlorid mit Alkalichlorid bildet, ohne daß ein Zusatz von Alkalihydroxid erforderlich ist. Ein Vorteil des erfindungsgemäßen Verfahrens gegenüber dem Stand der Technik besteht darin, daß einerseits nur geringe Mengen Abwasser anfallen, die andererseits lediglich einen niedrigen Natriumchloridgehalt aufweisen. Das Wertprodukt fällt in hoher Ausbeute an und weist lediglich untergeordnete Mengen Alkalichlorid auf. So wird das Natriumsalz der 3-Sulfobenzoesäure in Ausbeuten > 90 % gebildet. Es enthält weniger als 5 Gew.-% Natriumchlorid.

Ein weiterer Vorteil des erfindungsgemäßen Verfahrens ergibt sich aus der Wiederverwendbarkeit sowohl der als Filtrat bei der Umsetzung von 3-Benzoesäuresulfochlorid anfallenden als auch der als Waschlösung verwendeten Alkalichlorid-Lösung. So läßt sich das Filtrat gegebenenfalls mit gebrauchter Waschlösung auf die benötigte Menge aufstocken, wobei erforderlichenfalls durch Zusatz von Alkalihydroxid, Alkalihydrogencarbonat und/oder Alkalicarbonat ein pH-Wert von 3 bis 8, insbesondere 4 bis 6 unter Neutralisation von noch vorhandener Salzsäure eingestellt wird. Das derart behandelte Filtrat kann für einen weiteren Ansatz verwendet werden. Dadurch verringert sich die je Ansatz als Abwasser anfallende Menge wäßriger Alkalichloridlösung.

Die nachfolgenden Beispiele belegen die Erfindung, ohne sie zu beschränken.

### Experimenteller Teil

### Beispiel 1

### Herstellung von 3-Sulfobenzoesäure Natriumsalz

In einem 1 l-Vierhalskolben, bestückt mit Rührer, Innenthermometer und Rückflußkühler, werden 360 g Natriumchlorid-Lösung 26,4 %ig, die aus 95,0 g Natriumchlorid und 265,0 g Wasser hergestellt wurde, und 332 g 3-Benzoesulfochlorid tel quel (Wassergehalt: 39,2 %) (entsprechend 201,8 g (0,92 mol) 3-Benzoesäuresulfochlorid 100 %ig) eingetragen. Die Reaktionsmischung wird auf 100°C erwärmt und 45 Minuten bei dieser Temperatur nachgerührt. Danach läßt man das Gemisch innerhalb etwa 1 Stunde auf 40°C abkühlen. Anschließend kühlt man mit einem Eisbad auf < 5°C ab und rührt noch 30 Minuten bei dieser Temperatur nach. Das ausgefallene Produkt wird abgesaugt und in 2 Portionen mit 256 g 5°C kalter Natriumchlorid-Lösung 5%ig, die aus 12,8 g Natriumchlorid und 243,2 g Wasser hergestellt wurde, gewaschen. Der Filterkuchen wird bei 100°C/100 Torr (13,16 kPa) getrocknet. Man erhält 196,3 g 3-Sulfobenzoesäure Natriumsalz tel quel (Gehalt 95 %; Natriumchlorid-Gehalt: 3,9 %), (entsprechend 186,5 g (0,83 mol) 3-Sulfobenzoesäure Natriumsalz 100 %ig). Die Ausbeute beträgt 90,9 % d.Th.. Es fallen 345 g Mutterlauge und 318 g Waschlauge an.

### Beispiel 2

### Herstellung von 3-Sulfobenzoesäure Natriumsalz

In einem 2 l-Vierhalskolben, bestückt mit Rührer, Innenthermometer und Rückflußkühler, werden 600 g Natriumchlorid-Lösung 26,4 %ig und 290 g 3-Benzoesulfochlorid tel quel (Wassergehalt: 29,9 %) (entsprechend 203,3 g (0,92 mol) 3-Benzoesäuresulfochlorid 100 %ig) eingetragen. Die Reaktionsmischung wird auf 100°C erwärmt und 1 Stunde bei dieser Temperatur nachgerührt. Danach läßt man das Gemisch innerhalb etwa 1 Stunde auf 40°C abkühlen. Anschließend kühlt man mit einem Eisbad auf < 5°C ab und rührt noch 30 Minuten bei dieser Temperatur nach. Das ausgefallene Produkt wird abgesaugt und in 4 Portionen mit 180 g 5°C kalter Natriumchlorid-Lösung 5%ig gewaschen. Der Filterkuchen wird bei 100°C/100 Torr (13,16 kPa) getrocknet. Man erhält 200,0 g 3-Sulfobenzoesäure Natriumsalz tel quel (Gehalt 94,5 %; Natriumchlorid-Gehalt: 4,1 %), dies entspricht 189,0 g (0,84 mol) 3-Sulfobenzoesäure Natriumsalz 100 %ig gerechnet. Die Ausbeute beträgt 91,5 % d.Th..

### Beispiel 3

### Herstellung von 3-Sulfobenzoesäure Natriumsalz unter Rückführung der Mutterlauge

345 g Mutterlauge aus Beispiel 1 werden mit ca. 60 g Natriumhydroxid auf pH 4 gestellt. 300 ml dieser Lösung werden mit 264,5 g 3-Benzoesäuresulfochlorid (Wassergehalt: 25,7 %), (entsprechend 196,5 g (0,89 mol) 3-Benzoesäuresulfochlorid 100 %ig) eingetragen. Die Reaktionsmischung wird auf 100°C erwärmt und 45 Minuten bei dieser Temperatur nachgerührt. Danach läßt man das Gemisch innerhalb etwa 1 Stunde auf 40°C abkühlen. Anschließend kühlt man mit einem Eisbad auf < 5°C ab und rührt noch 30 Minuten bei dieser Temperatur nach. Das ausgefallene Produkt wird abgesaugt und in 2 Portionen mit 256 g 5°C kalter Natriumchlorid-Lösung 5%ig, die aus 13,8 g Natriumchlorid und 243,2 g Wasser hergestellt wurde, gewaschen. Der Filterkuchen wird bei 100°C/100 Torr (13,16 kPa) getrocknet. Man erhält 192,6 g 3-Sulfobenzoesäure Natriumsalz tel quel (Gehalt 96 %; Natriumchlorid-Gehalt: 3,2 %), (entsprechend 184,9 g (0,83 mol) 3-Sulfobenzoesäure Natriumsalz 100 %ig). Die Ausbeute beträgt 92,6 % d.Th..

### Vergleichsbeispiel 1

### Herstellung von 3-Sulfobenzoesäure-Natriumsalz gemäß US 4 358 410

In einem 1l-Vierhalskolben, bestückt mit Rührer, Innenthermometer und Rückflußkühler, werden 122,1 g (1,0 mol) Benzoesäure bei 125 bis 130°C geschmolzen. Dann werden innerhalb von 30 Minuten 405 g Oleum eingetragen. Anschließend erhitzt man das Reaktionsgemisch auf 130°C und rührt 1 Stunde bei dieser Temperatur nach. Das Reaktionsgemisch wird abgekühlt und innerhalb von 15 Minuten in 1800 g (= 1500 ml) Natriumchloridlösung 26,4 %ig einlaufen gelassen. Die Reaktionsmischung wird auf 80°C bis 85°C erwärmt. Danach läßt man das Gemisch unter Rühren auf 50°C abkühlen. Anschließend wird mit einem Eisbad auf < 5°C abgekühlt und noch 30 Minuten bei dieser Temperatur nachgerührt. Das ausgefallene Produkt wird abgesaugt und mit 256 (= 250 ml) 5°C kalter Natriumchloridlösung 5%ig, in 2 Portionen gewaschen. Man erhält nach Trocknen 204,4 g 3-Sulfobenzoesäure-Natriumsalz tel quel (Gehalt 96 %; Natriumchlorid-Gehalt: 3 %), entsprechend 196,3 g (0,88 mol) 3-Sulfobenzoesäure-Natriumsalz 100 %ig. Es fallen 2342 g Abwasser an.

### Vergleichsbeispiel 2

### Herstellung von 3-Benzoesäuresulfochlorid gemäß DE 39 19 840, Beispiel 16

In einem 2l-Vierhalskolben, bestückt mit Rührer, Innenthermometer, Rückflußkühler mit Gasableitung und Tropftrichter, werden 349,5 g = 199,7 ml (3,0 mol) Chlorsulfonsäure und 20,0 g Schwefelsäure 96 %ig bei 25°C vorgelegt und 1,0 g Amidosulfonsäure zugesetzt. Anschließend werden in diese Mischung 122,1 g (1,0 mol) Benzoesäure eingetragen. Die Reaktionsmischung wird innerhalb von 3 Stunden auf 120°C aufgeheizt und bis zum Ende der HCl-Entwicklung nachgerührt (ca. 30 Minuten). Danach wird auf 70°C abgekühlt und es werden innerhalb von 2 Stunden 119,0 g (1,0 mol) Thionylchlorid zugetropft. Die Reaktionslösung wird nun auf 80°C erwärmt und ca. 30 Minuten bis zum Ende der Gasentwicklung nachgerührt. Das Gemisch wird in 1000 g Eiswasser bei 10°C innerhalb von 2 Stunden getropft. Das ausgefallene Produkt wird abgesaugt und mit 500 g Eiswasser gewaschen. Man erhält 321,2 g 3-Benzoesäuresulfochlorid tel quel (Wassergehalt: 33,9 %; Chlorwert: 10,8 %), entsprechend 212,3 g (0,96 mol) 3-Benzoesäuresulfochlorid 100 %ig. Es fallen 1115 g Mutterlauge und 538 g Waschwasser an.

Wie die in der nachfolgenden Tabelle zusammengefaßte Gegenüberstellung zeigt, führt die Kombination des in der DE 39 19 840 beschriebenen Verfahrens mit dem erfindungsgemäßen Verfahren im Vergleich zum Stand der Technik, repräsentiert durch die US 4 358 410, zu einer Verringerung nicht nur der Abwassermenge, sondern auch zu einer Abnahme der im Abwasser enthaltenden Abfallstoffe.

**Tabelle**

| Vergleich der Abwassermengen und Inhaltsstoffe des Abwassers, das bei der Herstellung von 1 mol 3-Sulfobenzoesäure Natriumsalz, ausgehend jeweils von Benzoesäure, anfällt. | | | | |
|---|---|---|---|---|
| | 1* 3-Sulfobenzoesäure Natriumsalz gemäß US 4 358 410 | 2* Benzoesäuresulfochlorid gemäß DE 39 19 840 | 3* NaCl-Hydrolyse gemäß Beispiel 1 | 4* Summe aus 2* und 3* |
| Abwassermenge: | 2674 g | 1888 g + | 382 g | 2270 g |
| enthält | | | | |
| H₂SO₄ | 371 g | 247 g + | - | 247 g |
| NaCl | 476 g | - + | 40 | 40 g |
| HCl | 41 g | 83 g + | - | 83 g |

| | | | | |
|---|---|---|---|---|
| 1* = Vergleichsbeispiel 1; | | | | |
| 2* = Vergleichsbeispiel 2; | | | | |
| 3* = Beispiel 1; | | | | |
| 4* = Summe von 2* und 3*. | | | | |

## Patentansprüche

1. Verfahren zur Herstellung von Alkalisalzen der 3-Sulfobenzoesäure, dadurch gekennzeichnet, daß man 3-Benzoesäuresulfochlorid mit einer wäßrigen Alkalichloridlösung versetzt, das Gemisch erhitzt, nach Umsetzung auf ≦ 15°C abkühlt und das auskristallisierte Alkalisalz der 3-Sulfobenzoesäure abtrennt.

2. Verfahren nach Anspruch 1, dadurch gekennzeichnet, daß man als Alkalichlorid Natriumchlorid oder Kaliumchlorid, insbesondere Natriumchlorid einsetzt.

3. Verfahren nach Anspruch 1 oder 2, dadurch gekennzeichnet daß man eine wäßrige Lösung mit 15 bis 30, insbesondere 18 bis 28, bevorzugt 22 bis 27 Gew.-% Alkalichlorid einsetzt.

4. Verfahren nach einem oder mehreren der Ansprüche 1 bis 3, dadurch gekennzeichnet, daß man eine wäßrige Lösung mit 22 bis 26,4 Gew.-% Natriumchlorid einsetzt.

5. Verfahren nach einem oder mehreren der Ansprüche 1 bis 4, dadurch gekennzeichnet, daß man 0,5 bis 10, insbesondere 0,7 bis 4, bevorzugt 0,8 bis 2,5 Gew.-Teile wäßrige Alkalichloridlösung, insbesondere Natriumchloridlösung je Gew.-Teil 3-Benzoesäuresulfochlorid einsetzt.

6. Verfahren nach einem oder mehreren der Ansprüche 1 bis 5, dadurch gekennzeichnet, daß man das Gemisch auf eine Temperatur von 80°C bis Siedetemperatur des Gemisches, insbesondere 90°C bis Siedetemperatur des Gemisches erhitzt.

7. Verfahren nach einem oder mehreren der Ansprüche 1 bis 5, dadurch gekennzeichnet, daß man das Gemisch nach Umsetzung zunächst auf 60 bis 40, insbesondere 50 bis 40°C und anschließend auf ≦ 15°C, insbesondere ≦ 10°C, bevorzugt ≦ 5°C abkühlt.

8. Verfahren nach einem oder mehreren der Ansprüche 1 bis 7, dadurch gekennzeichnet, daß man das Alkalisalz der 3-Sulfobenzoesäure mit einer wäßrigen, 1 bis 10, insbesondere 3 bis 8 Gew.-% Alkalichlorid, vorzugsweise Natriumchlorid enthaltenden Lösung, die eine Temperatur ≦ 15, insbesondere ≦ 10, bevorzugt ≦ 5°C aufweist, wäscht.

9. Verfahren nach einem oder mehreren der Ansprüche 1 bis 8, dadurch gekennzeichnet, daß man die anfallende wäßrige Alkalichloridlösung, die gegebenenfalls durch Zusatz von Alkalihydroxid, Alkalihydrogencarbonat oder Alkalicarbonat auf einen pH-Wert von 3 bis 8, insbesondere 4 bis 6 eingestellt wird, wieder in das Verfahren einsetzt.

## Claims

1. A process for preparing alkali metal 3-sulfobenzoates, which comprises admixing 3-(chlorosulfonyl)benzoic acid with an aqueous alkali metal chloride solution, heating the mixture and, after reaction, cooling to ≦ 15°C and separating off the crystallized alkali metal 3-sulfobenzoate.

2. The process as claimed in claim 1, wherein the alkali metal chloride used is sodium chloride or potassium chloride, in particular sodium chloride.

3. The process as claimed in claim 1 or 2, wherein an aqueous solution containing from 15 to 30, in particular from 18 to 28 and preferably from 22 to 27 % by weight of alkali metal chloride is used.

4. The process as claimed in one or more of claims 1 to 3, wherein an aqueous solution containing from 22 to 26.4 % by weight of sodium chloride is used.

5. The process as claimed in one or more of claims 1 to 4, wherein from 0.5 to 10, in particular from 0.7 to 4 and preferably from 0.8 to 2.5 parts by weight of aqueous alkali metal chloride solution, in particular sodium chloride solution, are used per part by weight of 3-(chlorosulfonyl)benzoic acid.

6. The process as claimed in one or more of claims 1 to 5, wherein the mixture is heated to a temperature of from 80°C to the boiling point of the mixture, in particular from 90°C to the boiling point of the mixture.

7. The process as claimed in one or more of claims 1 to 5, wherein the mixture after reaction is cooled firstly to from 60 to 40°C, in particular from 50 to 40°C and subsequently to ≦ 15°C, in particular ≦ 10°C and preferably ≦ 5°C.

8. The process as claimed in one or more of claims 1 to 7, wherein the alkali metal 3-sulfobenzoate is washed with an aqueous solution containing from 1 to 10, in particular from 3 to 8 % by weight of alkali metal chloride, preferably sodium chloride, and having a temperature of ≦ 15°C, in particular ≦ 10°C and preferably ≦ 5°C.

9. The process as claimed in one or more of claims 1 to 8, wherein the aqueous alkali metal chloride solution produced, which has, if necessary, its pH adjusted to from 3 to 8, in particular from 4 to 6, by addition of alkali metal hydroxide, alkali metal hydrogen carbonate or alkali metal carbonate, is reused in the process.

## Revendications

1. Procédé de préparation de sels de métaux alcalins de l'acide 3-sulfobenzoïque, caractérisé en ce que l'on ajoute une solution aqueuse d'un chlorure de métal alcalin à de l'acide 3-chlorosulfonylbenzoïque, on chauffe le mélange, on le refroidit après la réaction à une température ≦ 15°C et on sépare le sel de métal alcalin de l'acide 3-sulfobenzoïque cristallisé.

2. Procédé selon la revendication 1, caractérisé en ce que, comme chlorure de métal alcalin, on utilise du chlorure de sodium ou du chlorure de potassium, en particulier du chlorure de sodium.

3. Procédé selon la revendication 1 ou 2, caractérisé en ce que l'on utilise une solution aqueuse contenant de 15 à 30, en particulier de 18 à 28, de préférence de 22 à 27 % en masse de chlorure de métal alcalin.

4. Procédé selon l'une ou plusieurs des revendications 1 à 3, caractérisé en ce que l'on utilise une solution aqueuse contenant de 22 à 26,4 % en masse de chlorure de sodium.

5. Procédé selon l'une ou plusieurs des revendications 1 à 4, caractérisé en ce que l'on utilise de 0,5 à 10, en particulier de 0,7 à 4, de préférence de 0,8 à 2,5 parties en masse de solution aqueuse de chlorure de métal alcalin, en particulier de solution de chlorure de sodium, pour une partie en masse d'acide 3-chlorosulfonylbenzoïque.

6. Procédé selon l'une ou plusieurs des revendications 1 à 5, caractérisé en ce que l'on chauffe le mélange à une température comprise entre 80°C et la température d'ébullition du mélange, en particulier entre 90°C et la température d'ébullition du mélange.

7. Procédé selon l'une ou plusieurs des revendications 1 à 5, caractérisé en ce que, après la réaction, on refroidit le mélange d'abord à une température de 60 à 40°C, en particulier de 50 à 40°C, puis à une température inférieure ou égale à 15°C, en particulier inférieure ou égale à 10°C, de préférence inférieure ou égale à 5°C.

8. Procédé selon l'une ou plusieurs des revendications 1 à 7, caractérisé en ce qu'on lave le sel de métal alcalin de l'acide 3-sulfobenzoïque avec une solution aqueuse contenant de 1 à 10, en particulier de 3 à 8 % en masse de chlorure de métal alcalin, de préférence de chlorure de sodium, ayant une température inférieure ou égale à 15°C, en particulier inférieure ou égale à 10°C, de préférence inférieure ou égale à 5°C.

9. Procédé selon l'une ou plusieurs des revendications 1 à 8, caractérisé en ce que l'on réutilise dans le procédé la solution aqueuse de chlorure de métal alcalin produite, que l'on amène éventuellement à un pH compris entre 3 et 8, en particulier entre 4 et 6, en ajoutant un hydroxyde de métal alcalin, un hydrogénocarbonate de métal alcalin ou un carbonate de métal alcalin.
